# EUROPEAN PATENT APPLICATION

(11) **EP 2 316 444 A2**
(43) Date of publication of application: **04.05.2011**
(21) Application number: 11155163.6
(22) Date of filing: 23.01.2007
(51) Int. Cl.: A61K 31/165, A61K 31/192, A61K 31/194, A61K 31/20, A61K 31/203, A61K 31/232, A61K 31/4436, A61K 45/06, A61P 17/00, A61P 17/06, A61P 17/10

(54) **Use of alkanedicaroxylic acids and retinoids for the treatment of rosacea and other inflammatory skin diseases**

(30) Priority: 23.01.2006 DE 102006004804
(62) Divisional of application: 07703154.0
(71) Applicant: Intendis GmbH, 10589 Berlin (DE)
(72) Inventor: Zollner, Thomas, 12161, Berlin (DE); Johannsen, Soenke, 10115, Berlin (DE)
(74) Representative: Gebauer, Olaf

(57) **Abstract**

In order to make available another therapeutic possibility for rosacea the use of at least one α,ω-n-alkanedicarboxylic acid with 7 to 13 carbon atoms or its esters and at least one retinoid for producing a pharmaceutical preparation is proposed. The suggested formulation is also effective in treatment of other inflammatory skin diseases such as psoriasis, atopic dermatitis and common acne.

## Description

The invention relates to the use of alkanedicarboxylic acids and retinoids for producing a pharmaceutical preparation for treatment of rosacea and other inflammatory skin diseases.

### Prior Art

It is known from DE 28 17 133 to use α,ω-n-alkanedicarboxylic acids with 7 to 13 carbon atoms and their esters that can be decomposed by skin enzymes for treatment of hyperpigmentary dermatoses or dermal hyperpigmentations. Furthermore, EP 0 336 880 A2 describes a pharmaceutical composition that contains azelaic acid and that is used to treat various age-induced changes of the facial skin. The use of azelaic acid alone (as the single active ingredient) against inflammatory dermatoses, such as, for example, acne and rosacea, is also included in the prior art.

EP 0 443 413 B1 discloses a pharmaceutical composition for anti-photoaging and depigmenting skin treatment that contains azelaic acid and tretinoic acid as active ingredients. Use of this combination of active ingredients for anti-inflammatory purposes is not described.

Further prior art can be found in:
DE 69400428 T2
CN 1185951 A
US 2002/0155180 A1
US 2003/077301 A1
US 2005/0169948 A1
WO 99/581104 A1
WO 2005/115546 A2
US 4661519

### Detailed description of the Invention

The object of this invention is to make available another therapeutic possibility for rosacea and other inflammatory skin diseases such as psoriasis, atopic dermatitis, and common acne, or a corresponding pharmaceutical preparation.

This object is achieved by the features of the claims.

According to the invention, the use of
- at least one α,ω-n-alkanedicarboxylic acid with 7 to 13 carbon atoms or its esters and
- at least one retinoid
is implemented for producing a pharmaceutical preparation for treatment of inflammatory skin diseases.

Preferred are α,ω-n-alkanedicarboxylic acids or their esters that are selected from the following group:
pimelic acid, suberic acid, azelaic acid, sebacic acid, 1,9-nonanedicarboxylic acid, 1,10-decanedicarboxylic acid and 1,11-undecanedicarboxylic acid.

Azelaic acid or its esters is more preferred, and azelaic acid is most preferred.

Alkyl groups of 1 to 5 carbon atoms, independently of one another, can be used at the α- and ω-positions as ester groups. Alkyl groups can be methyl, ethyl, propyl, butyl, or pentyl in straight or branched form.

Retinoids from the following group are preferred:
tretinoin, isotretinoin, motretinide, etretinate, adapalene, bexarotene and tazarotene.
Tretinoin and isotretinoin and adapalene, are more preferred, and tretinoin is most preferred.

A pharmaceutical preparation according to the invention or the use of the above-mentioned active ingredients according to the invention is especially suited for skin diseases that have inflammatory components and that are described below:
- Rosacea is an initially chronically hyperemic (rosacea I), later chronically inflammatory (rosacea II and II) disease of the face. It can be attributed to increased vascular reactions and inflammatory processes with infiltration of leukocytes and release of inflammatory mediators. It is characterized by persistent erythema having acute inflammatory phases with the formation of edemas, papules and pustules. Successful treatment of rosacea is difficult. The composition according to the invention is suited to exerting a beneficial effect both on the inflammatory component (papules and pustules) and on the vascular components of rosacea (erythema and telangiectases).
- Psoriasis is an inflammatory, chronic, hyperproliferative skin disease. The lesions are sharply delineated, thick, erythematous spots with proliferating white desquamation. Conventionally, the elbow, knee, facial skin, genitals and gluteal folds are affected. The composition according to the invention is especially well suited to exerting a beneficial effect on inflammatory lesions.
- Atopic dermatitis/neurodermatitis is a skin disease with symptoms that are essentially equivalent to those of other eczemas, inflammatory skin changes such as redness, desquamation, small blisters, papules, and nodules, accompanied by generally irritating pruritus, being the most important criteria. Moreover, very dry skin is characteristic. The use of the active ingredients according to the invention acts especially on the inflammatory portions of the disease.
- Acne is an anomaly of the sebaceous glands with inflammatory papules, pustules and cysts and with non-inflammatory blackheads. In most cases, it affects juveniles and young adults. The composition according to the invention is suited to exerting an altogether favorable effect on acne, a clear reduction of papules, pustules and cysts being noted.

The use of the aforementioned active ingredients according to the invention in combination in the treatment of inflammatory skin diseases, as briefly described above for individual clinical pictures, shows, surprisingly enough to one skilled in the relevant art, significant therapeutic effectiveness that distinctly exceeds that of the two individual substances.

Test results that indicate an additive or synergistic effect of a combination therapy are unknown to date.

The effectiveness of the combination of active ingredients for inflammatory skin diseases was demonstrated using a popular model for pharmacological evaluation of the anti-inflammatory potential of topically or systemically applied test substances. To do this, an inflammatory reaction, i.e., irritative contact dermatitis, is induced on the ears of mice by means of croton oil, and the test substance is applied. This test is explained in more detail below within the framework of the examples.

Use according to the invention comprises preventive and therapeutic measures. The components of the pharmaceutical preparation that is to be produced for use according to the invention can be administered at the same time and/or spatially in one dose; furthermore, they can be applied separately physically and/or in time from one another.

For therapeutic effects, the suitable dose is different and depends on, for example, the concentration of the individual elements in the pharmaceutical preparation, the host, the type of administration, and the type and severity of the conditions that are to be treated.

Preferred concentrations of the α,ω-n-alkanedicarboxylic acids, especially of azelaic acid, are in the range of 2 to 40% (percent by weight), more preferably 5 to 30%, and most preferably 10 20%.

A concentration of retinoids, especially tretinoin, of 0.005% to 0.2% (percent by weight), is preferred, 0.025% to 0.1% is more preferred, and 0.04% to 0.06% is most preferred.

Furthermore it is preferred that besides the two active components α,ω-n-alkanedicarboxylic acids and retinoid no further active component is present. It is especially preferred that the preparations according to the invention do not contain squalane, nicotinamide, dexamethasone (or other glucocorticoids or corticosteroids), dimethyl sulfone, metronidazole (or other antbiotics).

The pharmaceutical preparation produced for the use according to the invention can be processed into conventional topical application forms with additives and/or vehicles that are conventional in galenical pharmacy according to methods that are known in the art. A composition according to the invention together with at least one physiologically compatible pharmacological adjuvant or vehicle is preferred. Pharmacological adjuvants and vehicles are described in Remington's Pharmaceutical Science, 15th Ed., Mack Publishing Company, Easton, Pennsylvania (1980).

The pharmaceutical preparation can be administered in topical treatment in any conventional manner by converting the active ingredients with suitable additives into the desired application form, such as, for example, solutions, lotions, creams, ointments, pastes or gels. The gel is preferred for acne and rosacea. The lotions, creams, ointments and gels can be produced in the conventional manner using conventional emulsifiers (Kirk Othmer: Encyclopedia of Chemical Technology, 3rd Ed., 1979, John Wiley & Sons, New York, Vol. 8, pp. 900-930).

Moreover, keratolytic agents, such as, for example, salicylic acid, vitamin A acid, resorcinol, phenol, cresol and the like may be added to the pharmaceutical preparation (although this is not a preferred embodiment).

Humectant factors (hydro complexes), such as, for example, propylene glycol, glycerol, polyethylene glycols or amino acid mixtures, puroba oil (= jojoba oil), vitamins, preferably vitamins A and E, vital complexes (such as, for example, placental extracts), enzymes, herbal extracts (such as, for example, hamamelis extract or chamomile extract) or proteins (such as, for example, collagen) may be used as hydrophilic and/or lipophilic additives. Hydrocarbons, such as, for example, vaseline, paraffins, or stearin, or waxes, such as, for example, beeswax, are suited as the oily phase or fatty phase in an oil/water emulsion. Suitable oil/water emulsifiers are, for example, stearyl alcohol, polyoxyethylene stearate (such as, for example, MTRJ®, complex emulsifiers (such as, for example, Amphoterin® and sorbitan fatty acid esters (such as, for example, Tween 80^{®}) or carboxyvinyl polymerizates (such as, for example, Carbopol^{®}). The aqueous phase can additionally contain buffer substances, such as, for example, the disodium salt of ethylenediamine-N,N,N',N'-tetraacetic acid and preservatives, such as benzoic acid, chlorquinaldol, parabene or benzalkonium chloride.

According to one especially preferred embodiment for treatment of acne and rosacea, the bioavailability of the two active ingredients is increased by using the following pharmaceutical vehicles:
Triacyl glycerides, propylene glycol, polysorbates, polyacrylic acid, and soy lecithin in an aqueous phase comprising water and salts, the composition constituting a hydrogel.

### Examples

The invention is explained in more detail below using diagrams/examples. Here:
Figure 1 shows a bar chart for presentation of edema formation on the ear of a mouse,
Figure 2 shows a bar chart for presentation of neutrophil infiltration and
Figure 3 shows a bar chart for presentation of granulocyte infiltration.

The induction of an inflammatory reaction (irritative contact dermatitis) is a proven model for pharmacological evaluation of the anti-inflammatory potential of topically or systemically administered test substances (Trancik and Lowe (1985) Evaluation of Topical Nonsteroidal Anti-Inflammatory Agents, Models in Dermatology, H. Maibach and N. J. Lowe. Basel, Karger: 35-42).

Croton oil is applied to the ears of mice to carry out the study; this leads to an acute, inflammatory reaction with edema and infiltration of primarily polymorphonuclear granulocytes that reaches its peak within 24 hours. Test substance can be applied here either preventively, i.e., before application of the inflammatory stimulus, or simultaneously with croton oil.

In this test, 1% croton oil in ethanol/isopropyl myristate as the vehicle was applied at time t = 0 h. The vehicle alone acted as the vehicle control. The substances azelaic acid (10 - 20%) and tretinoin (0.05%) were applied to the mouse ear either alone or as a combination, simultaneously with the irritant croton oil, likewise dissolved in ethanol/isopropyl myristate. The animals were sacrificed 24 hours after the eczema was triggered, and the ear weight was determined as a measure of inflammation and edema formation. In addition, the elastase and peroxidase parameters were determined from the ear homogenate. The number of animals was basically n = 10/group.

Elastase is a largely neutrophil-specific enzyme and thus a measure of the number of infiltrated neutrophilic granulocytes. Neutrophilic granulocytes play an important part in inflammatory processes of the skin and are detectable in the skin in a considerably increased number in rosacea, acne, psoriasis vulgaris and atopic eczema. Peroxidase is expressed by all granulocytes, in addition also by monocytes. It is used for measuring the infiltrated granulocytes and monocytes. Like neutrophils, granulocytes and monocytes/macrophages are also involved in inflammatory processes of the skin and can be repeatedly detected in the infiltrate of the aforementioned diseases.

The tests comprise application of the following substances to the ears of mice:
- Vehicle (ethanol/isopropyl myristate) Reference No.: 1
- Croton oil Reference No.: 2
- Azelaic acid (10%) Reference No.: 3
- Azelaic acid (15%) Reference No.: 4
- Azelaic acid (20%) Reference No.: 5
- Tretinoin (0.05%) Reference No.: 6
- Azelaic acid (10%) and tretinoin (0.05%) Reference no.: 7
- Azelaic acid (15%) and tretinoin (0.05%) Reference No.: 8
- Azelaic acid (20%) and tretinoin (0.05%) Reference No.: 9

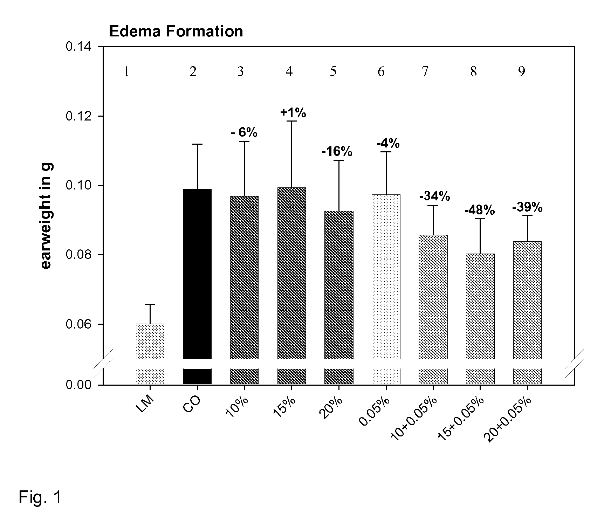

The bar chart according to Figure 1 shows the formation of an edema on the mouse ear after application of the respective substance. The average weight of a mouse ear after application (1) of the vehicle is 0.06 g. The application of croton oil (2) causes an increase in the weight of the ear, due to edema and cell infiltration as indications of inflammation, by which the basis for determining the percentage deviation after application of the test substance is given.

The application of azelaic acid takes place with concentrations of 10% (3), 15% (4) and 20% (5). Here, no significant reduction of the edema can be observed; in contrast, at a concentration of 15%, even an increase can be noted. The application of tretinoin in a concentration of 0.05% (6) does not lead to any significant reduction of edema or inflammation of the ear measured on the ear weight. The combined application of azelaic acid and tretinoin, however, surprisingly shows a considerable reduction of the edema. Thus, the application of azelaic acid 10% and tretinoin (0.05%) (7), of azelaic acid (15%) and tretinoin (0.05%) (8) and azelaic acid (20%) and tretinoin (0.05%) (9) leads to improvements of edemas by 34%, 48% and 39%, respectively. Thus, a synergistic mechanism is clearly demonstrated in the administration of the two therapeutic agents.

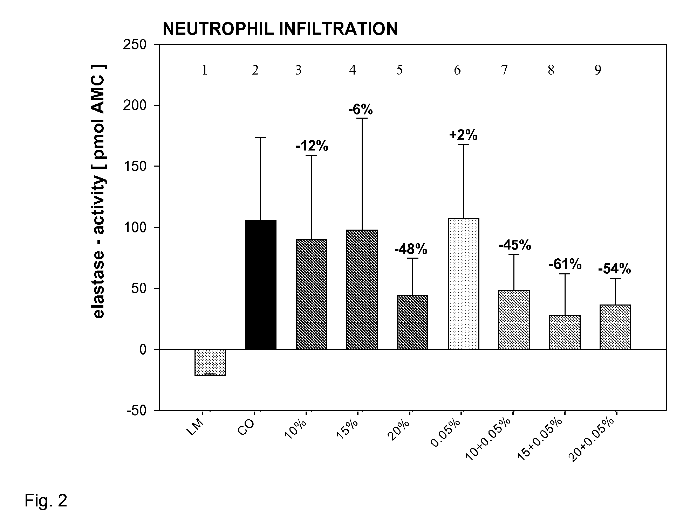

A corresponding finding also arises from the determination of the elastase activity as a measure of the inhibition of skin infiltration of neutrophils. Croton oil causes an increase in the concentration of neutrophilic elastase, due to cell infiltration by neutrophils within the framework of inflammation. The corresponding values for the elastase activity are shown in Figure 2 as a bar chart. These values reflect the result that corresponds to Figure 1, i.e., the values cannot be significantly influenced by azelaic acid application alone in a concentration range of 10-15% (3, 4). Neutrophilic elastase was reduced only by 20% azelaic acid (5). A single application of tretinoin 0.05% (6) in the same manner as that of azelaic acid 10-15% (3, 4) did not lead to a decrease of the elastase concentration in the ear homogenate. Surprisingly enough, however, the combination of azelaic acid 10-15% with tretinoin 0.05% (7, 8) led to a significant reduction of ear inflammation, measured on the elastase concentration in the ear homogenate. This in turn confirms a synergistic mechanism in the combination of the two therapeutic agents. This could no longer be detected for 20% azelaic acid (9) since in this concentration of the substance, a very pronounced inhibition of elastase was already detectable, which then could no longer be further increased by additional application of tretinoin.

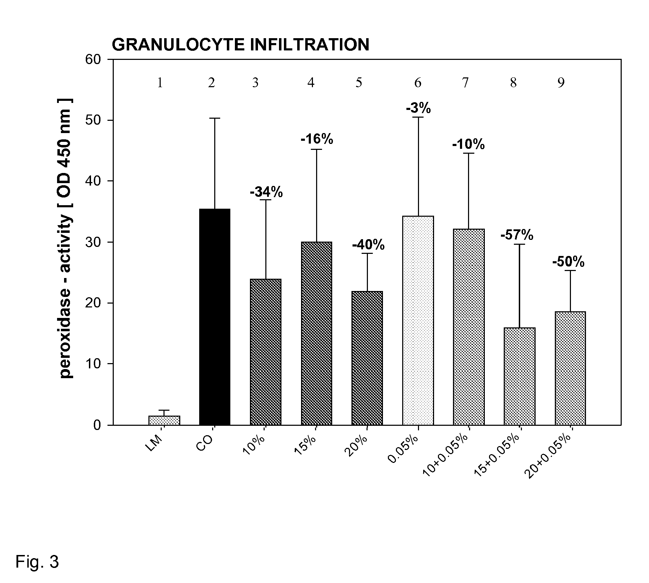

Figure 3 shows, likewise in the form of a bar chart, peroxidase activity as a measure of the inhibition of skin infiltration of monocytes and granulocytes. Croton oil causes an increase in the concentration of peroxidase, due to cell infiltration by granulocytes and monocytes within the framework of inflammation. The latter could already be influenced in 2 out of 3 applied azelaic acid concentrations (3, 5). A single application of tretinoin 0.05% (6), however, did not lead to a significant decrease of peroxidase concentration. However, in the combined administration of the two therapeutic agents (7, 8, 9), especially at a concentration of 15-20% (8, 9), in turn a significant inhibition of this parameter was induced, which indicates a synergistic effect.

The results of this study are summarized below in Table 1.

**Table 1**

| | | **Edema formation** | **Inhibition [%] of Skin infiltration * of** | |
|---|---|---|---|---|
| **Trigger** | **Treatment** | **Weight change in %** | **Neutrophils** | **Granulocytes & Monocytes** |
| | | | Elastase Activity | |
| | | | | (Myelo-PeroxidaseActivity) |
| Vehicle | Vehicle | - | - | - |
| Croton oil | Vehicle (Negative control) | 0 | 0 | 0 |
| Croton oil | Azelaic acid (10%) | -6 | -12 | -34 |
| Croton oil | Azelaic acid (15%) | 1 | -6 | -16 |
| Croton oil | Azelaic acid (20%) | -16 | -48 | -40 |
| Croton oil | Tretinoin(0.05%) | -4 | 2 | -3 |
| Croton oil | Azelaic acid (10%) + Tretinoin (0.05%) | -34 | -45 | -10 |
| Croton oil | Azelaic acid (15%) + Tretinoin (0.05%) | -48 | -61 | -57 |
| Croton oil | Azelaic acid (20%) + Tretinoin (0.05%) | -39 | -54 | -50 |

In summary, it can be stated that -- unexpectedly for one skilled in the art -- a combination of tretinoin (0.05%) and azelaic acid (10-20%) in ethanol/isopropyl myristate compared to the vehicle was significantly therapeutically effective, while the two individual substances tretinoin (0.05%) and azelaic acid (10-20%) did not manifest any equally great therapeutic effects compared to the vehicle.

Leukocyte infiltration into the mouse skin inflamed by croton oil is significantly inhibited by combined treatment with tretinoin and azelaic acid compared to treatment with the vehicle, while the individual agents were not as effective.

The effectiveness included, i.a., the infiltration of leukocytes measured as peroxidase activity in the skin as well as the infiltration of neutrophilic granulocytes measured as elastase activity in the skin (Table 1).

The infiltration of this cell population into the skin and the release of pro inflammatory substances are involved in the pathogenesis of a series of inflammatory diseases. These diseases include, i.a., rosacea, acne, allergic and irritative contact dermatitides, atopic dermatitis, and psoriasis (Braum - Falco et al. (1995) Dermatologie und Venerologie [Dermatology and Venerology]. Heidelberg, Springer).

### Further studies

Based on these promising results further studies confirm the effectiveness of formulations according to the invention in treatment ofrosacea, allergic and irritative contact dermatitis, atopic dermatitis, psoriasis, and acne.

## Claims

1. Use of at least one α,ω-n-alkanedicarboxylic acid with 7 to 13 carbon atoms or its esters and at least one retinoid for producing a pharmaceutical preparation for treatment of rosacea.

2. Use according to claim 1, wherein at least one α,ω-n-alkanedicarboxylic acid is selected from the group:
pimelic acid, suberic acid, azelaic acid, sebacic acid, 1,9-nonanedicarboxylic acid, 1,10-decanedicarboxylic acid and 1,11-undecanedicarboxylic acid.

3. Use according to claim 2, wherein at least one α,ω-n-alkanedicarboxylic acid is azelaic acid.

4. Use according to one of the preceding claims, wherein alkyl groups of 1 to 5 carbon atoms independently of one another are present at the α- and ω-positions as ester groups.

5. Use according to claim 4, wherein the alkyl groups are methyl, ethyl, propyl, butyl, or pentyl in straight or branched form.

6. Use according to one of the preceding claims, wherein at least one retinoid is selected from the group: tretinoin, isotretinoin, motretinide, etretinate, adapalene, bexarotene, and tazarotene.

7. Use according to claim 6, wherein at least one retinoid is tretinoin.

8. Use according to one of the preceding claims, wherein at least one α,ω-n-alkanedicarboxylic acid or its esters in a concentration of 2 to 40% is present.

9. Use according to claim 8, wherein at least one α,ω-n-alkanedicarboxylic acid or its esters in a concentration of 5 to 30% is present.

10. Use according to claim 8, wherein at least one α,ω-n-alkanedicarboxylic acid or its esters in a concentration of 10 to 20% is present.

11. Use according to one of the preceding claims, wherein at least one retinoid in a concentration of 0.005% to 0.2% is present.

12. Use according to claim 11, wherein at least one retinoid in a concentration of 0.025% to 0.1 % is present.

13. Use according to claim 11, wherein at least one retinoid in a concentration of 0.04% to 0.06% is present.

14. Use according to one of the preceding claims, wherein the use takes place with pharmaceutical additives and/or vehicles.

15. Use according to claim 14, wherein the use comprises the following pharmaceutical vehicles: triacyl glycerides, propylene glycol, polysorbates, polyacrylic acid, and soy lecithin in an aqueous phase, comprising water and salts.

16. Use according to one of the preceding claims, wherein the use does not comprise the use of any further pharaceutically active compound.

17. Use of at least one α,ω-n-alkanedicarboxylic acid with 7 to 13 carbon atoms or its esters and at least one retinoid for producing a pharmaceutical preparation for treatment of inflammatory skin disease.

18. Use of at least one α,ω-n-alkanedicarboxylic acid with 7 to 13 carbon atoms or its esters and at least one retinoid for producing a pharmaceutical preparation for treatment of psoriasis, atopic dermatitis, or common acne.

19. Use according to claim 17 or 18, wherein at least one α,ω-n-alkanedicarboxylic acid is selected from the group:
pimelic acid, suberic acid, azelaic acid, sebacic acid, 1,9-nonanedicarboxylic acid, 1,10-decanedicarboxylic acid and 1,11-undecanedicarboxylic acid.

20. Use according to claim 17 or 18, wherein at least one α,ω-n-alkanedicarboxylic acid is azelaic acid.

21. Use according to to claim 17 or 18, wherein alkyl groups of 1 to 5 carbon atoms independently of one another are present at the α- and ω-positions as ester groups.

22. Use according to to claim 17 or 18, wherein the alkyl groups are methyl, ethyl, propyl, butyl, or pentyl in straight or branched form.

23. Use according to claim 17 or 18, wherein at least one retinoid is selected from the group:
tretinoin, isotretinoin, motretinide, etretinate, adapalene, bexarotene, and tazarotene.

24. Use according to to claim 17 or 18, wherein at least one retinoid is tretinoin.
